# EUROPEAN PATENT APPLICATION

(11) **EP 4 831 159 A1**
(43) Date of publication of application: **28.10.2026**
(21) Application number: 26171209.5
(22) Date of filing: 08.04.2026
(51) Int. Cl.: C07C 305/10, C07C 309/31, C07C 309/42, C11D 1/22, C11D 1/34

(54) **ALKYLATED ARYL SURFACTANTS**

(30) Priority: 14.04.2025 US 202563788285 P
(71) Applicant: Pilot Chemical Corp., West Chester, OH 45069 (US)
(72) Inventor: LEAR, Jeremy Michael, West Chester, 45069 (US); DALTON, Emily Lace, West Chester, 45069 (US); MANKA, John Stephen, West Chester, 45069 (US)
(74) Representative: Forresters IP LLP

(57) **Abstract**

Surfactants formed from alkylated aryl compounds are disclosed. The surfactants exhibit desirable performance and have improved safety profiles. Methods of making and using the surfactants are further described.

## Description

### BACKGROUND

Certain applications including emulsion polymerizations, household, industrial, and institutional ("HI&I") compositions, agricultural compositions, oilfield compositions, and other specialty applications require the inclusion of high-performance surfactants to act as an emulsifier, wetting agent, dispersant, or detergent. High-performance surfactants can exhibit one or more of high charge density, excellent solubility, stability in acidic, alkaline, and oxidizing systems, and tolerance to hard water and high salt concentrations that facilitate their use in challenging or specialty applications. Although known high-performance surfactants perform satisfactorily, there is a continuing need for additional high-performance surfactants having desirable physical properties and which exhibit improved safety profiles and performance and/or which can be manufactured using environmentally friendly technology.

### SUMMARY

According to one embodiment, a surfactant composition can include an anionic surfactant represented by the structure of Formula I: 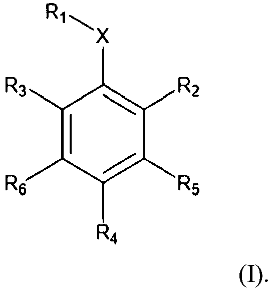 wherein:
X can be S or O;
R₁ can include H or a non-aromatic alkyl group;
R₂, R₃, R₄, R₅, and R₆ each independently can include H, a C6 to C28 linear or branched alkyl group, SO₃ , SO₃H; PO₃H₂, PO₃H, or PO₃²⁻ and
wherein:
at least one of R₂, R₃, R₄, R₅, and R₆ includes SO₃⁻, SO₃H; PO₃H₂, PO₃H, or PO₃²⁻; and
at least one of R₂, R₃, R₄, R₅, and R₆ includes a C6 to C28 linear or branched alkyl group.

According to another embodiment, a method of forming an alkylated sulfonic acid surfactant includes providing an oxyphenyl or sulfophenyl group and an alkyl group; performing an alkylation reaction with the alkyl group and the oxyphenyl or sulfophenyl group to form an alkylated oxyphenyl or sulfophenyl group; and sulfonating the alkylated oxyphenyl or sulfophenyl group to form an alkylated sulfonic acid surfactant.

According to another embodiment, a method of forming an alkylated anisole surfactant includes providing anisole and an alkyl group, performing an alkylation reaction with the alkyl group and the anisole to form an alkylated anisole; and sulfonating the alkylated anisole to form an alkylated anisole surfactant.

### BRIEF-DESCRIPTION OF THE DRAWINGS

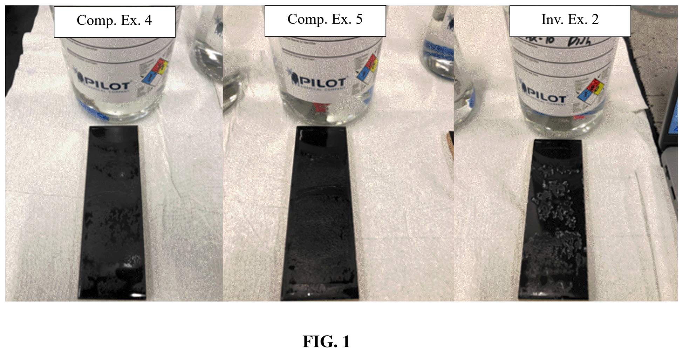

FIG. 1 depicts a photograph of test strips after being cleaned with various example surfactant compositions to demonstrate degreasing performance.

### DETAILED DESCRIPTION

As will be described herein, high-performance surfactants are disclosed which exhibit improved safety and performance compared to known high-performance surfactants. Generally, the described surfactants can be alkyl aryl surfactants, or related derivatives thereof, having the structure depicted in Formula I: 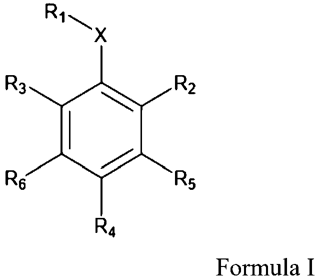 wherein:
X comprises O or S;
R₁ comprises H or a non-aromatic alkyl group;
R₂, R₃, R₄, R₅, and R₆ each independently can include H, a C6 to C28 linear or branched alkyl group, SO₃⁻, SO₃H; PO₃H₂, PO₃H, or PO₃²⁻ and
wherein:
at least one of R₂, R₃, R₄, R₅, and R₆ includes SO₃⁻, SO₃H; PO₃H₂, PO₃H, or PO₃²⁻; and
at least one of R₂, R₃, R₄, R₅, and R₆ comprise a C6 to C28 linear or branched alkyl group.

In certain embodiments, the described surfactants can be alkyl aryl alkoxy sulfonate surfactants wherein X in Formula I is oxygen. Also referred to alkyl anisole surfactants, such surfactants can exhibit desirable physical properties including low viscosity, low color, and excellent cleaning and emulsion polymerization performance. In alternative embodiments, X can be sulfur. Both oxygen and sulfur can increase the reactivity of the aryl ring due to resonance activation. The aryl rings of the described surfactants further include hydrogen or preferably a non-aromatic alkyl group attached via an ether or sulfide bond at R₁ through X. In certain embodiments, R₁ can be a methyl group.

Generally, the described surfactants can include one, two, or three alkyl substituents at positions R₂ to R₅ in Formula I. In certain embodiments, each such alkyl substituent can independently be a linear or branched C6 to C28 alkyl. In certain embodiments, such alkyl substituents can be individually, or in combination, any of C8 linear, C10 linear, C12 linear and/or C12 branched alkyl substituents, which exhibit the desired performance for respective applications. As can be appreciated, other alkyl substituents can be used for applications requiring tailored performance characteristics. In certain embodiments, the surfactants described herein can include a combination of a linear and a branched alkyl substituents. Alternatively, in certain embodiments, each alkyl substituent can be the same.

The alkyl substituents can be selected based on the desired properties and intended use of the described surfactants. For example, surfactants including C10 linear alkyl substituents can exhibit excellent cleaning properties and can be useful for household, institutional, and industrial ("HI&I") products. Additionally, C10 linear alkyl substituents can also be useful for emulsion polymerizations as C10 linear alkyl substituents can exhibit superior emulsion polymerization properties including reduced amounts of fouling and grit and improved emulsion stability.

As will be appreciated, the number and position of the alkyl substituents can affect the properties of the described surfactant. As an example, the critical micelle concentration ("CMC") of the described surfactants can vary depending upon the number and type of the alkyl substituents. Additionally, for certain applications, the performance of the described surfactants can vary depending upon the number of alkyl substituents with a blend of about 85% mono alkylated surfactant and about 15% bis alkylated surfactant performing significantly better than a similar composition formed of about 95% mono alkylated surfactant and about 5% bis alkylated surfactant for reducing fouling/grit in an emulsion polymerization.

Although the described surfactants can include the alkyl substituents at any position R₂ to R₅, certain positions on the aryl ring have been found to result in surfactants that exhibit improved properties. For example, in some embodiments, advantageous surfactants have been developed having a single alkyl substituent is in the para position (e.g., R₄) relative to X in Formula I. In such embodiments, the sulfonic acid or sulfonate group can be in the ortho (R₂ or R₃) position. In other advantageous embodiments, if there are two alkyl substituents, the second substituent can be located at the remaining para position.

The aryl ring further includes a hydrophilic group to provide the amphiphilic properties to the surfactants described herein. The hydrophilic group can be sulfonic acid, sulfonate, phosphoric acid, or phosphonate and can be in any position around the aryl ring. In certain embodiments, the hydrophilic group can be in the ortho or para positions. In certain embodiments, the hydrophilic group can be in the para position.

As can be appreciated, ring positioning of the alkyl substituents and the hydrophilic group can be influenced by resonance activation of the ring by X. Such activation of the aryl ring can provide additional benefits. For example, such activation can ease manufacturing, improve color, and can reduce viscosity of the surfactants described herein.

With respect to manufacturing and color, activation of the aryl ring enhances alkylation and sulfonation processes. For example, such activation can enable alkylation and sulfonation to proceed under milder conditions which can enable the described surfactants to exhibit lower color, without bleaching or additional processing, as reduced amounts of sulfonation compounds are required and reduced temperatures can be used. Many applications require, or prefer, mild coloration of high-performance surfactants.

The viscosity of the described surfactants was also found to be unexpectedly lower than similar diphenyl oxide disulfonate and alkylbenzene sulfonate surfactants having similar substituent alkyl groups. Without being bound by theory, it is believed that intra molecular interaction of the lone pair on the oxygen of the alkoxy group with the sulfonic acid / sulfonate group can reduce the intermolecular interaction preventing association of molecules which can effectively reduce the apparent molecular weight in solution and thus keeps the viscosity lower than in the diphenyl oxide and alkylbenzene based surfactants. As can be appreciated, lower viscosity can be useful for formulations incorporating the described surfactants and for mixing and transportation of the surfactants. Similar effects are expected when sulfur replaces oxygen.

In certain embodiments, surfactant compositions including the described surfactants can exhibit improved performance if a blend of surfactants having different numbers of alkyl substituents are included. For instance, the CMC of a surfactant composition including about 87% of a surfactant having a single alkyl substituents and substantially the remainder being a surfactant having two alkyl substituents, can have a CMC of about 0.62 g/L compared to a CMC of about 1.48 g/L for a similar composition having about 95% of the composition comprise a surfactant having a single alkyl substituent and substantially the remainder being a surfactant having two alkyl substituents.

In certain embodiments, surfactant compositions can comprise about 80% to about 90% of a described surfactant having single alkyl substituents and about 10% to about 20% of the surfactant composition comprise a described surfactant including two alkyl substituents or any percentages between such ranges. In certain embodiments, surfactant compositions can include about 83% to about 89% of a described surfactant having a single alkyl substituent and about 11% to about 17% of a described surfactant including two alkyl substituents. In certain embodiments, about 85% to 88% of a surfactant composition can be a surfactant including a single alkyl substituent and the substantial remainder can be a surfactant having two alkyl substituents. In such embodiments, a small portion of the surfactant composition can include about 1% or less of a surfactant including three alkyl substituents. In certain embodiments, the surfactant compositions can include a blend of surfactants having alkyl substituents of different length, such as, for example, a blend of C8 to C12 linear alkyl substituents.

In certain embodiments, the surfactants described herein can be neutralized to form a salt. Generally, any suitable cation can be used as the counterion to the hydrophilic group such as ammonium, aminium, or mono or divalent metals of group I or group II such as sodium, potassium, magnesium, or calcium. In certain such embodiments, the associated cation can be sodium or an ammonium cation as such cations can exhibit lower viscosity and greater solubility than the alternative cations.

Generally, the described surfactants and surfactant compositions can exhibit desirable properties in comparison to known high-performance surfactants. For example, when compared to sodium dodecyl diphenyl oxide disulfonate, the surfactants described herein can perform equivalently, or better, than sodium dodecyl diphenyl oxide disulfonate at applications such as cleaning and emulsion polymerization.

In certain embodiments, the described surfactants and/or surfactant compositions can exhibit a lower viscosity than comparable high-performance surfactants. The lower apparent viscosity is believed to be caused by the arrangement of the hydrophilic group near the electronegative group on the aryl ring. Lower viscosity enables the high-performance surfactants described herein to be used in more applications with less mixing or dilution, and with easier handling.

The surfactants and surfactant compositions described herein can exhibit improved safety profiles than known high-performance surfactants based on computer studies and the safety of the underlying components.

In certain embodiments, the described surfactants can be bio-based as all of the components used to form the described surfactants can have renewable feedstocks. For example, bio-derived linear olefins can be used for the alkylation. In certain embodiments, the described surfactants can be formed only from renewable resources. Advantageously, the described surfactants can biodegrade faster and more readily than alkyldiphenyloxide disulfonate surfactants or alkylbenzene sulfonate surfactants.

### Methods of Making

The described surfactants can generally be formed by 1) alkylation of aryl; followed by 2) sulfonation or phosphonylation to form the described surfactants.

### Alkylation

To prepare the surfactants described herein, a suitable aryl can be selected and then alkylated. For example, for anisole-based surfactants, anisole can be alkylated to form the desired alkylated anisole prior to sulfonation or phosphonylation. As can be appreciated however, other aryl compounds can be selected in other embodiments.

Generally, any suitable alkylation process can be used. For example, in certain embodiments, a suitable alkylation process can be a Friedel-Crafts alkylation. In such embodiments, the desired alkyl can be provided as an alkyl olefin and then attached to the aryl (such as anisole) in the presence of a suitable catalyst such as a strong Lewis acid. Generally, any catalyst known for use in Friedel-Crafts alkylation can be used including Lewis acids and solid acid catalysts. For example, suitable catalysts can include FeCl₃, FeBr₃, BF₃, SbCl₅, AlBr₃, AlCl₃, GaCl₃, GaCl₂, SbF₅, MoCl₅ (ii) InCl₃, LnBr₃, AlCl₃-CH₃NO₂, SbF₅-CH₃NO₂ (iii) BCl₃, SnCl₄, TiCl₄, TiBr₄, FeCl₂, and zeolites. In certain embodiments, the catalyst can be AlCl₃.

Alternatively, alkylation can be performed using alkyl olefins and Brønsted acids as a catalyst. Suitable Brønsted acids can include trifluoromethane sulfonic acid in hexafluoroisopropanol, superacids such as trifluoromethyl sulfonic acid (triflic acid), acidic cation-exchange resins, and certain metal halides.

Although alkylation using alkyl olefins is preferred, similar alkylation reactions can also be performed using alkylhalides or alkyl alcohols.

### Sulfonation or Phosphonylation

Alkylated aryls can be sulfonated or phosphonated to form the described surfactants. Generally, any known sulfonation process can be used including SO₂/SO₃ cold sulfonation processes, air SO₃ processes, sulfuric acid processes, oleum sulfonation processes, chlorosulfonic acid processes, and pyridine sulfonate complex processes. In certain embodiments, SO₂/SO₃ cold sulfonation processes can be preferred. Similarly, any phosphonylation method can be used as known in the art including photochemical methods and directed lithiation methods.

### Optional Manufacturing Steps

As can be appreciated, any number of additional manufacturing steps can be performed including purification, neutralization, and blending steps as known in the art. For example, distillation of fractional separation can be used to remove unreacted components and side products. In certain embodiments, the surfactants described herein can be neutralized to form a surfactant salt by addition of an appropriate ion such as ammonium or a group I or group II metal ion.

In certain embodiments, surfactant compositions can be formed by blending more than one surfactant described herein together. In yet other embodiments, a surfactant composition can be inherently formed from the reaction steps without requiring blending due to, for example, the alkylation step producing a blend of mono and di-alkylated aryls.

In certain embodiments, the aryl, such as anisole, can be prepared from starting reagents as known in the art. For example, anisole can be prepared from phenol by methylation using methyl iodide or methyl chloride, potassium carbonate, and DMF; by methylation using methyl sulfate; and by methylation using methylcarbonate, methyl halides, methanol, dimethyl carbonates, and dimethyl ether. Alternatively, other aryls can be formed using alkylation of phenol with alkyl phenol using diazo alkanes. In certain embodiments, the Williamson Ether synthesis can be used. In other embodiments, the aryl can be commercially obtained.

As can be appreciated, the Williamson Ether synthesis can used to form ethers by the reaction of an alkoxide (an alcohol with a hydrogen replaced by a metal) with an alkyl halide where an alkoxide ion displaces a halide ion from an alkyl halide, forming a new C-O bond resulting in an ether and a salt. The reaction can proceed via an SN2 mechanism, making it well suited for the reaction of primary and secondary alkyl halides with an alkoxide to give an ether. The alkoxide (or aryloxide) can be primary and secondary. Tertiary alkoxides tend to give elimination reactions because of steric hindrance. The alkylating agent (alkyl halide), on the other hand is most preferably primary. Secondary alkylating agents can also react, but tertiary ones are usually too prone to side reactions to be of practical use. The leaving group is most often a halide, or a sulfonate ester synthesized for the purpose of the reaction.

### Applications

The surfactants and surfactant compositions described herein can be utilized in numerous applications typically requiring use of high-performance surfactants. For example, the surfactants described herein can be used for emulsion polymerizations, HI&I products, oilfield compositions including enhanced oil recovery compositions, electroplating compositions, textile and pulp and paper products, inks and toners, agricultural applications including soil remediating and soil cleanup compositions, and battery and capacitor applications. Examples of HI&I products that can include the surfactants described herein can include cleaning products such as dish/laundry detergents, all-purpose cleaners and bathroom tub and tile cleaners as well as personal care products such as shampoos, body washes, lotions, and cleansers. Generally, the surfactants described herein can be used as the primary surfactant in such applications or can be included as a secondary surfactant.

### Examples

### Emulsion Polymerization

The described surfactants and surfactant compositions were evaluated to determine their ability to perform a latex emulsion polymerization that requires a high-performance surfactant. Surfactants affect monomer solubilization, particle size distribution, polymerization rate, and latex stability of an emulsion polymerization. Surfactants can also affect monomer mixture/ratios, agitation speeds/type, temperature, and salinity.

Inventive surfactants and surfactant compositions evaluated in the latex emulsion polymerization include Inventive Example 1, a blend including 88.5% mono C8 linear anisole sulfonate and 11.5% bis C8 linear anisole sulfonate; Inventive Example 2, a blend including 87.7% mono C10 linear anisole sulfonate, and 12.3% bis C10 linear anisole sulfonate; Inventive Example 3, a blend including 94.2% mono C10 linear anisole sulfonate, and 5.8% bis C10 linear anisole sulfonate; Inventive Example 4, a blend including 88.6% mono C12 linear anisole sulfonate and 11.4% bis C12 linear anisole sulfonate; and Inventive Example 5, a blend including 89.8% mono branched C12 anisole sulfonate and 10.2% bis branched C12 anisole sulfonate. Comparative surfactants included Comparative Example 1, sodium C12 branched diphenyl oxide disulfonate; Comparative Example 2, sodium C10 linear diphenyl oxide disulfonate; and Comparative Example 3, sodium C12 linear diphenyl oxide disulfonate. The weight percentage of sodium chloride, sodium sulfonate, and the pH of Inventive Examples 1, 2, 4, and 5 are depicted in Table 1.

**TABLE 1**

| **Example** | **Wt % NaCl** | **Wt% Na₂SO₄** | **pH (10% active)** |
|---|---|---|---|
| Inventive Example 1 | 0.0043 | 0.7718 | 10.2 |
| Inventive Example 2 | 0.0073 | 0.4096 | 10.8 |
| Inventive Example 4 | 0.0080 | 0.3425 | 10.4 |
| Inventive Example 5 | 0.0056 | 0.5609 | 11.0 |

The latex emulsion polymerization process was performed using a using a semi-continuous seed polymerization process over 3 hours. In the process, the seed was the initial reactor charge (water + evaluated surfactant), monomer pre-emulsion (monomers + water + evaluated surfactant), and initiator. The monomers used in the latex emulsion polymerization evaluation included styrene and butyl acrylate. Ammonium persulfate was used as an initiator.

The pre-emulsion was prepared by slowly adding the monomers to a 1.27 parts per hundred monomer ("pphm") surfactant solution. This pre-emulsion and an aqueous initiator solution were pumped into the reactor over the course of the reaction. The latex was cooled before filtering with a stainless steel 100 mesh to collect the final product. The latex was then analyzed for fouling, solid content, viscosity, and particle size.

The latex emulsion process was evaluated for emulsion stability, fouling, grit, solids, viscosity, particle size and dispersity, latex foaming, and latex shear stability:
**Emulsion Stability:** Observe emulsion over course of reaction (3 hours). Look for separation of monomer from emulsion. It can be as little as no separation, small amounts of separation with monomer droplets, or as inches of monomer separated from emulsion (would appear like clear liquid on top of emulsion), or complete separation of monomer and water.
**Fouling:** fouling is a thin layer of polymer deposited on the reactor equipment. Visual rating of the degree of fouling on the reactor wall and agitator blade: clean, slight, slight-medium, medium, heavy. Typical fouling ratings of industrial process are slight or slight-medium. When the latex is unstable, the particles might be coagulated into "chunks" or "balls" that might hinder the agitation and the reaction might have to be stopped.
**Grit:** Grit is aggregate of latex particles suspended in the latex. The finished latex was cooled and filtered through 100-micron stainless steel mesh. The filtrate was washed with water then dried in the 50⁰C oven over night and the weight was determined by gravimetric method. The weight was reported as ppm (dry/dry) on the theoretical weight of the non-volatile materials charged to the reactor during the course of the reaction.
**Solids:** Solids content is the weight percentage of non-volatile materials on the total weight of latex. Solids content was measured by gravimetric method (moisture balance, JML EP program: 0.5g sample, 175°C for 35 minutes)
**Viscosity:** viscosity was measured by the Brookfield DV-I+ with spindle #2 at 100 rpm Spindle number may need to be changed depending on viscosity.
**Particle size and dispersity.** Particle size and distribution impact viscosity, water resistance, film clarity, stability, and shear rheology. These properties were measured using dynamic light scattering (supplied by Microtrac, Inc.). 0.5 grams of latex is diluted to 50 grams with DI water.
**Latex foaming.** A 35% latex solution is prepared by diluting 35 grams of latex to 50 grams with DI water. The latex is then shaken using a handshaker for 2 minutes. The latex is poured into a graduated cylinder and the foam height is monitored over time.

The results of evaluating the disclosed surfactants and surfactant compositions are depicted in Table 2. Fouling/Grit, Emulsion Stability, Particle Size, and Dispersity were considered the most important properties for evaluating the surfactants as they are the most challenging parameters influenced by a surfactant for an emulsion polymerization. Fouling/Grit, Emulsion Stability, Latex Foam, and Electrolyte Stability were rated on a scale of 1 to 5 with 1 indicating poor results, 2 indicating fair results, 3 indicating good results, 4 indicating very good results and 5 indicating superior results. Ratings of 3 or higher or typically considered acceptable.

**TABLE 2**

| **Example** | **Inv. 1** | **Inv. 2** | **Inv. 3** | **Inv. 4** | **Inv. 5** | **Comp. 1** | **Comp. 2** | **Comp. 3** |
|---|---|---|---|---|---|---|---|---|
| Fouling / Grit (1-5) | 4 | 5 | 4 | 4 | 3 | 5 | 5 | 5 |
| Emulsion Stability (1-5) | 3 | 5 | 5 | 4 | 4 | 4 | 3 | 3 |
| Particle Size (nm) | 114 | 101 | 85 | 76 | 96 | 112 | 103 | 90 |
| Dispersity | 0.14 | 0.13 | 0.13 | 0.15 | 0.14 | 0.09 | 0.10 | 0.11 |
| Latex Foam (1-5) | 4 | 5 | 5 | 4 | 4 | 4 | 3 | 4 |
| CMC (g/L) | 0.43 | 0.62 | 1.48 | 0.25 | 1.49 | 0.36 | 0.26 | 0.16 |
| Electrolyte Stability (1-5) | 3 | 3 | 3 | 3 | 3 | 3 | 2 | 2 |

As depicted in Table 2, Inventive Examples 1-4 matched, or exceeded, the performance of the comparative examples for the most critical emulsion polymerization properties with Inventive Example 2 performing extremely well. It is generally understood that all high-performance surfactants can make small latex particles but differences in surfactant can have a large impact on fouling, CMC, and latex foam.

### Cleaning

To evaluate the ability of the described surfactants to be used for high-performance cleaning applications, an exemplary dish cleaning product was formulated as depicted in Table 3. The pH was adjusted to 8-8.5. The degreasing capability of Inventive Example 2 was compared to sodium C14-16 alpha olefin sulfonate (Comparative Example 4) and sodium linear alkylbenzene sulfonate (Comparative Example 5). Both comparative surfactants are used in HI&I cleaning products.

For evaluation, 3" by 2" black tiles were washed with tap water, isopropyl alcohol, and deionized water and then allowed to air dry. A gloss reader was then used to measure the gloss reading on the blank cleaned tiles (clean gloss). A paint roller was then saturated with vegetable shortening and 0.1-0.15g of vegetable shortening was placed on a tile. The paint roller was used to spread shortening over the tile and a gloss measurement recorded using the gloss reader (soiled gloss). 300g of each exemplary dish cleaning product was placed in a 400 mL beaker and placed in an oven set to 43 °C to equilibrate for at least 1 hour. Soiled tiles were then placed in the beaker for 5 minutes before being removed, dunked in room temperature tap water twice, placed on a paper towel for 5 minutes. The tiles were then placed back in the beakers for a second duration of 5 minutes before being dunked in room temperature tap water twice. The tiles were then allowed to air dry before a gloss reading was performed (dunked gloss). All measurements were performed in triplicate.

Shortening removal efficiency was calculated as the difference of the average dunked gloss with the average soiled gloss divided by the difference between the average clean gloss and the average soiled gloss and then multiplied by 100.

**TABLE 3**

| **Component** | **Wt.% Active** |
|---|---|
| Surfactant | 0.5 |
| Sodium Citrate | 0.5 |
| Water | Q.S. |

Inventive Example 2 exhibited superior degreasing performance compared to both comparative surfactants as depicted in FIG. 1. Specifically, FIG. 1 depicts test strips covered in shortening and then degreased with the dish cleaning product depicted in Table 4. As indicated in FIG. 2, Inventive Example 2 removed more of the shortening than either of the Comparative Examples. The shortening removal efficiency of Inventive Example 2 was about 40% versus about 20% and 7.5% for Comparative Examples 4 and 5 respectively.

### Physical Properties

The physical properties of Inventive Examples 1 to 5 were evaluated. The measured physical properties include rotational viscometry as measured using a 62 spindle at 100 rpm measured at 25 °C, the CMC, the surface tension, Drave's wetting, and the Ross-Miles foam height. The physical properties are depicted in Table 3.

**TABLE 3**

| **Inventive Example** | **1** | **2** | **3** | **4** | **5** |
|---|---|---|---|---|---|
| **Surfactant Viscosity (Spindle:62, 100 RPM @25 ±1 °C** | 81.3 cPs | 65.3 cPs | 62.4 cPs | 147.5 cPs | 45.4 cPs |
| **CMC** | 0.43 g/L | 0.62 g/L | 1.48 g/L | 0.25 g/L | 1.49 g/L |
| **Surface tension (mN,m)** | 30.93 | 33.21 | 33.51 | 33.98 | 31.10 |
| **Drave's wetting (sec)** | 3 | 3 | 2.5 | 3.5 | 4 |
| **Ross-Miles Foam height (Initial, mm)** | 75 | 155 | 155 | 148 | 159 mm |
| **Ross-Miles Foam height (Final 5min, mm)** | 52 | 105 | 80 | 144 | 115 mm |

The dimensions and values disclosed herein are not to be understood as being strictly limited to the exact numerical values recited. Instead, unless otherwise specified, each such dimension is intended to mean both the recited value and a functionally equivalent range surrounding that value.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Every document cited herein, including any cross-referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in the document shall govern.

The foregoing description of embodiments and examples has been presented for purposes of description. It is not intended to be exhaustive or limiting to the forms described. Numerous modifications are possible in light of the above teachings. Some of those modifications have been discussed and others will be understood by those skilled in the art. The embodiments were chosen and described for illustration of various embodiments. The scope is, of course, not limited to the examples or embodiments set forth herein, but can be employed in any number of applications and equivalent articles by those of ordinary skill in the art. Rather it is hereby intended the scope be defined by the claims appended hereto.

It should be understood that certain aspects, features, structures, or characteristics of the various embodiments can be interchanged in whole or in part. Reference to certain embodiments mean that a particular aspect, feature, structure, or characteristic described in connection with certain embodiments can be included in at least one embodiment and may be interchanged with certain other embodiments. The appearances of the phrase "in certain embodiments" in various places in specification are not necessarily all referring to the same embodiment, nor are certain embodiments necessarily mutually exclusive of other certain embodiments. It should also be understood that the steps of the methods set forth herein are not necessarily required to be performed in the orders described, and the order of the steps of such methods should be understood to be merely exemplary. Likewise, additional steps can be included in such methods, and certain steps may be omitted or combined, in methods consistent with certain embodiments.
The invention may be defined by the following clauses:
1. A surfactant composition comprising an anionic surfactant represented by the structure of Formula I: 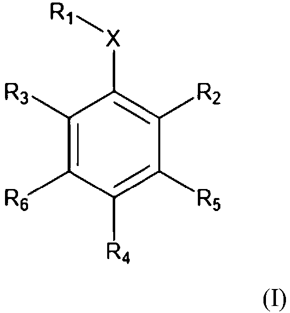 wherein:
   X comprises O or S;
   R₁ comprises H or a non-aromatic alkyl group;
   R₂, R₃, R₄, R₅, and R₆ each independently comprise H, a C6 to C28 linear or branched alkyl group, SO₃⁻, SO₃H;PO₃H₂, PO₃H, or PO₃²⁻ ;
   wherein:
   at least one of R₂, R₃, R₄, R₅, and R₆ comprises SO₃⁻; SO₃H; PO₃H₂, PO₃H, or PO₃²⁻; and
   at least one of R₂, R₃, R₄, R₅, and R₆ comprise a C6 to C28 linear or branched alkyl group.
2. The surfactant composition of clause 1, wherein the structure of Formula I comprises a salt.
3. The surfactant composition of clause 2, wherein a cation of the salt comprises ammonium, aminium, or a mono or divalent metal of group I or group II.
4. The surfactant composition of clause 2 or clause 3, wherein the cation comprises a sodium cation or an ammonium cation.
5. The surfactant composition of any preceding clause, wherein R₁ is H or CH₃.
6. The surfactant composition of any preceding clause, wherein only one of R₂, R₃, R₄, R₅, and R₆ comprises a C6 to C28 linear or branched alkyl group.
7. The surfactant composition of clause 6, wherein one of R₂, R₃, and R₄ comprises a linear C8 alkyl, a linear C10 alkyl, a linear C12 alkyl, or a branched C12 alkyl.
8. The surfactant composition of clause 6 or clause 7, wherein:
   R₁ is H or CH₃,
   R₂ is SO₃⁻ or SO₃H;
   R₄ is a linear C8 alkyl, C10 alkyl, a linear C12 alkyl, or a branched C12 alkyl; and
   R₃, R₅, and R₆ are H.
9. The surfactant composition of any preceding clause, wherein two of R₂, R₃, R₄, R₅, and R₆ comprise a C6 to C28 linear or branched alkyl group.
10. The surfactant composition of any preceding clause, wherein three of R₂, R₃, R₄, R₅, and R₆ comprise a C6 to C28 linear or branched alkyl group.
11. The surfactant composition of any preceding clause comprises two or more surfactants, each of the two or more surfactants comprising the structure of Formula (I).
12. The surfactant composition of clause 11, wherein about 80% to about 95wt% of the anionic surfactants in the surfactant composition comprise the structure of Formula I wherein only one of R₂, R₃, R₄, R₅, and R₆ comprise a C6 to C28 linear or branched alkyl group.
13. The surfactant composition of clause 12, wherein about 85wt% to about 98wt% of the anionic surfactants in the surfactant composition comprise the structure of Formula I wherein R₄ comprises a linear C8 alkyl, C10 alkyl, a linear C12 alkyl, or a branched C12 alkyl.
14. The surfactant composition of c clause 11, wherein about 87wt% of the anionic surfactants in the surfactant composition comprise the structure of Formula I wherein only one of R₂, R₃, R₄, R₅, and R₆ comprise a C6 to C28 linear or branched alkyl group.
15. The surfactant composition of any one of clauses 12 to 14, wherein a portion of the anionic surfactants in the surfactant composition comprise the structure of Formula I wherein two of R₂, R₃, and R₄ comprise a C6 to C28 linear or branched alkyl group.
16. The surfactant composition of any preceding clause has a critical micelle concentration of about 2 g/L or less.
17. The surfactant composition of any preceding clause exhibits a surface tension of about 35 mN/m or less.
18. The surfactant composition of any preceding clause exhibits a surface tension of about 28 mN/m to about 34 mN/m.
19. The surfactant composition of any preceding clause exhibits a Drave's wetting time of about 3 seconds to about 5 seconds.
20. The surfactant composition of any preceding clause is a substantially bio-derived surfactant composition.
21. An agricultural product comprising the surfactant composition of any preceding clause.
22. Use of the surfactant composition of any one of clauses 1 to 20 for an emulsion polymerization.
23. Use of the surfactant composition of any one of clauses 1 to 20 for cleaning products comprising home care and personal care; wherein the home care products comprise dish soap, laundry soap, all-purpose cleaners and bathroom tub and tile cleaners and personal care comprises shampoos and body wash.
24. Use of the surfactant composition of any one of clauses 1 to 20 for Enhanced Oil Recovery / Production / Oilfield products.
25. Use of the surfactant composition of any one of clauses 1 to 20 for electroplating.
26. Use of the surfactant composition of any one of clauses 1 to 20 for textiles and pulp and paper as a leveling agent.
27. Use of the surfactant composition of any one of clauses 1 to 20 for inks and toners.
28. Use of the surfactant composition of any one of clauses 1 to 20 for soil remediation / soil cleanup.
29. Use of the surfactant composition of any one of clauses 1 to 20 for use in lithium based and other battery applications.
30. A method of forming an alkylated sulfonic or phosphonic acid surfactant, comprising:
   providing an oxyphenyl or sulfophenyl group and an alkyl group;
   performing an alkylation reaction with the alkyl group and the oxyphenyl or sulfophenyl group to form an alkylated oxyphenyl or sulfophenyl group; and
   sulfonating the alkylated oxyphenyl or sulfophenyl group to form an alkylated sulfonic acid surfactant.
31. The method of clause 30, wherein the alkylation reaction comprises a Friedel-Crafts alkylation reaction and wherein the alkyl group comprises an olefin, alcohol or an alkyl halide.
32. The method of clause 31, wherein the Friedel-Crafts alkylation reaction uses aluminum chloride as a catalyst.
33. The method of clause 31, wherein the Friedel-Crafts alkylation reaction uses a Lewis Acid, Bronsted acid, or a solid acid catalyst as a catalyst.
34. The method of clause 30, wherein the etherification reaction comprises a Williamson Ether Synthesis.
35. The method of clause 30, wherein the etherification reaction comprises methylation with dimethylsulfate.
36. The method of clause 30, wherein the etherification reaction comprises methylation with methylcarbonate.
37. The method of any one of clauses 30 to 36, wherein the alkylated oxyphenyl or sulfophenyl group is sulfonated using a SO₂/SO₃ process.
38. The method of any one of clauses 30 to 36, wherein the alkylated oxyphenyl or sulfophenyl group is sulfonated using an air SO₃ process, an oleum sulfonation process, sulfuric acid sulfonation process, a chlorosulfonic acid process, or a pyridine sulfonate complex.
39. The method of any one of clauses 30 to 38, wherein the olefin comprises a C8 linear olefin, C10 linear olefin, a C12 linear olefin, or a C12 branched olefin.
40. A method of forming an alkylated anisole surfactant, comprising:
   providing anisole and an alkyl group;
   performing an alkylation reaction with the alkyl group and the anisole to form an alkylated anisole; and
   sulfonating the alkylated anisole to form an alkylated anisole surfactant.
41. The method of clause 40, wherein the olefin comprises

## Claims

1. A surfactant composition comprising an anionic surfactant represented by the structure of Formula I: 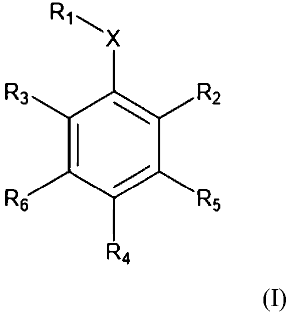 wherein:
X comprises O or S;
R₁ comprises H or a non-aromatic alkyl group;
R₂, R₃, R₄, R₅, and R₆ each independently comprise H, a C6 to C28 linear or branched alkyl group, SO₃⁻, SO₃H;PO₃H₂, PO₃H, or PO₃²⁻ ;
wherein:
at least one of R₂, R₃, R₄, R₅, and R₆ comprises SO₃⁻; SO₃H; PO₃H₂, PO₃H, or PO₃²⁻; and
at least one of R₂, R₃, R₄, R₅, and R₆ comprise a C6 to C28 linear or branched alkyl group.

2. The surfactant composition of claim 1, wherein the structure of Formula I comprises a salt of ammonium, aminium, or a mono or divalent metal of group I or group II.

3. The surfactant composition of any previous claim, wherein R₁ is H or CH₃.

4. The surfactant composition of any previous claim, wherein one or more of R₂, R₃, R₄, R₅, and R₆ independently comprise a C6 to C28 linear or branched alkyl group.

5. The surfactant composition of claim 4, wherein one of R₂, R₃, and R₄ comprises a linear C8 alkyl, a linear C10 alkyl, a linear C12 alkyl, or a branched C12 alkyl.

6. The surfactant composition of any previous claim, wherein:
R₁ is H or CH₃,
R₂ is SO₃⁻ or SO₃H;
R₄ is a linear C8 alkyl, C10 alkyl, a linear C12 alkyl, or a branched C12 alkyl; and
R₃, R₅, and R₆ are H.

7. The surfactant composition of any previous claim comprises two or more surfactants, each of the two or more surfactants comprising the structure of Formula (I).

8. The surfactant composition of claim 7, wherein about 80% to about 95wt% of the anionic surfactants in the surfactant composition comprise the structure of Formula I wherein only one of R₂, R₃, R₄, R₅, and R₆ comprise a C6 to C28 linear or branched alkyl group.

9. The surfactant composition of claim 8, wherein about 85wt% to about 98wt% of the anionic surfactants in the surfactant composition comprise the structure of Formula I wherein R₄ comprises a linear C8 alkyl, C10 alkyl, a linear C12 alkyl, or a branched C12 alkyl.

10. The surfactant composition of claim 7, wherein about 87wt% of the anionic surfactants in the surfactant composition comprise the structure of Formula I wherein only one of R₂, R₃, R₄, R₅, and R₆ comprise a C6 to C28 linear or branched alkyl group.

11. The surfactant composition of claims 8 to 10, wherein a portion of the anionic surfactants in the surfactant composition comprise the structure of Formula I wherein two of R₂, R₃, and R₄ comprise a C6 to C28 linear or branched alkyl group.

12. The surfactant composition of any previous claim has a critical micelle concentration of about 2 g/L or less.

13. The surfactant composition of any previous claim exhibits a surface tension of about 35 mN/m or less.

14. The surfactant composition of any previous claim exhibits a surface tension of about 28 mN/m to about 34 mN/m.

15. The surfactant composition of any previous claim exhibits a Drave's wetting time of about 3 seconds to about 5 seconds.
